# EUROPEAN PATENT APPLICATION

(11) **EP 2 865 350 A2**
(43) Date of publication of application: **29.04.2015**
(21) Application number: 14190445.8
(22) Date of filing: 27.10.2014
(51) Int. Cl.: A61B 18/14, A61B 18/00, A61B 18/12

(54) **Electrode ablation balloon catheter**

(30) Priority: 25.10.2013 US 201361895678 P; 02.10.2014 US 201414504548
(71) Applicant: Covidien LP, Mansfield, MA 02048 (US)
(72) Inventor: Jameson, Allen D., Sunnyvale, CA California 94086 (US); Bagley, Christopher L., Santa Clara, CA California 95054 (US); Huszar, Hillary K., Redwood City, CA California 94065 (US); Maguire, Mark A., Hillsborough, CA California 94010 (US); Utley, David S., Redwood City, CA California 94062 (US)
(74) Representative: Hoffmann Eitle

(57) **Abstract**

Methods, systems, and devices for providing treatment to a tissue in body lumens are described. The system may include a support shaft, an expansion member coupled with a distal portion of the support shaft, and an ablation structure wrapped around the expansion member less than a circumference of the expansion member configured to engage the body lumens with varying sizes. The ablation structure may include multiple separately wired or separately controlled longitudinal electrodes, longitudinal electrode zones, or both, such that each longitudinal electrode or longitudinal electrode zone may be selectively enabled or selectively disabled. The expansion member may include a single highly-compliant balloon, a single non-compliant balloon, multiple non-compliant balloons, or a multi-chambered non-compliant balloon.

## Description

### CROSS REFERENCE TO RELATED APPLICATION

The present application claims the benefit of and priority to U.S. Provisional Application Serial No. 61/895,678, filed on October 25, 2013, the entire contents of which are incorporated herein by reference.

### BACKGROUND

The human body has a number of internal body lumens or cavities located within, such as the differing parts of the gastro-intestinal tract, many of which have an inner lining or layer. Body lumens may include, for example, the esophagus, small and large intestines, stomach, remnant after bariatric surgery, rectum and anus. These inner linings may be susceptible to disease. In some cases, different ablation techniques have been utilized with respect to the inner lining in order to prevent the spread of disease to otherwise healthy tissue located nearby.

Internal body lumens may have different sizes with respect to each other or with respect to different patients. As a result, different sized devices may have been utilized to accommodate these different sized lumens. However, this may involve utilizing multiple devices such as multiple sizing and/or treatment devices, which may not be efficient or cost effective. In addition, prior approaches often lacked the flexibility to reduce or eliminate over ablation in body lumens of varying diameters.

There may thus be a need for systems, devices and methods that may overcome the above and/or other disadvantages of known systems, devices, and methods.

### SUMMARY

Methods, systems, and devices are described for providing treatment to a target site, such as a site within a body lumen. Systems may include an expansion member that may be coupled with a distal portion of a support shaft. An ablation structure with a circumference less than the circumference of the expansion member may be wrapped around the expansion member such that expanding the expansion member will engage body lumens of varying sizes. In some embodiments, the ablation structure includes a number of longitudinal electrode regions. In some instances, the ablation structure may have a circumference equal to about half the circumference of the expansion member. Upon expansion of the expansion member, the ablation structure will engage a portion of the circumference of the body lumen, resulting in partial circumferential ablation. The expansion member and attached ablation structure may then be rotated to one or more additional positions such that the unablated area or gap may be ablated. Over ablation due to electrode elements overlapping previously ablated tissue may be reduced and/or eliminated by switching on or switching off electrode regions.

For example, after a first ablation of a partial circumferential region of a body lumen, additional regions of the body lumen treatment area may be ablated by rotating and positioning the expansion member and attached ablation structure such that one end of the ablation structure is aligned with a border of a previously ablated area. The electrode regions of the ablation structure may then be switched on and enabled such that the additional regions are ablated. Depending, in part, on the circumference of the body lumen, one or more of the repositioning steps may include one or more end electrode regions or a portion of one or more end electrode regions overlapping a portion or portions of the previously ablated tissue. One or more of the end electrode regions may be switched off and/or remain disabled during ablation events where overlap conditions exist, such that over ablation of previously ablated tissue is reduced or eliminated. This process may be repeated one or more times until the desired portion of the circumference of the treatment site, in many cases the entire circumference of the treatment site, is ablated. The number of repositioning steps and the degree of overlap may depend, in part, on the size of the body lumen under treatment, the arc length of the ablation structure, and ablation structure positioning of one or more prior positioning steps.

The ablation structure may include multiple separately wired and/or separately controlled longitudinal electrode regions consisting of longitudinal electrodes, longitudinal electrode zones, or both, such that each longitudinal electrode or longitudinal electrode zone may be selectively enabled or selectively disabled. For purposes of this application, an electrode region means a defined radio frequency energy (RF) application area of an electrode that does not overlap with other defined RF energy application areas of an electrode. In some instances, electrode regions may be configured such that energy is delivered to the entire electrode region when activated. For purpose of this application, a longitudinal electrode zone means a defined portion of the surface area of a longitudinal electrode. In some instances, the area of one or more electrode zones extends for the full length of the electrode area and less than the full width of the electrode area. In some implementations, electrode elements are circumferentially oriented within one or more longitudinal electrode zones. An electrode zone may have a width greater than, less than, or equal to its length. In some instances, the ablation structure includes an electrode array, such as, for example, a bipolar electrode array. The ablation structure may include longitudinal electrodes of varying widths, longitudinal electrode zones of varying widths, or both.

A power source, such as an RF generator, may deliver energy to electrode regions over one or more RF channels. In some embodiments, each RF channel is associated with a single electrode region such that the there is a one to one relationship between the number of electrode regions and the number of RF channels provided by the power source. The power source may be communicatively coupled to an automated channel selection logic module and/or a manual channel selection interface. The manual channel selection interface may be directly coupled to the power source or operate external to the power source. An external switching mechanism may be communicatively coupled to the power source using established communication protocol such as I2C or SPI. In another embodiment, the switching mechanism may increase the number of electrode regions beyond the number of RF channels provided by the power source.

In addition to increasing the number of channels, the switching mechanism may also selectively enable and selectively disable electrode regions, thus controlling, in part, the arclength of the tissue ablated and reducing or eliminating over ablation of previously ablated tissue. The switching mechanism may include a circuit configured to re-route and/or block delivery of energy to electrode regions based on feedback or input from an operator and/or an automated selection logic module. The switching mechanism may be communicatively coupled to manual selection interface such as, for example, a button. In some implementations, this selection interface is located on the handle of the catheter. The selection interface may be a part of the switching circuit and may be configured to control which channels transmit energy. In another embodiment the selection detected by the selection interface may be sent to the power source.

The expansion member may include one or more non-compliant balloons configured to fold in a manner that avoids pinching of the ablation structure. For example, one or more non-compliant balloons may undergo a manufacturing or treatment process directed towards increasing stiffness or creating a specific conformation, such as, for example, concave electrode folds. This may be accomplished by, for example, heat shaping of the balloon, introduction of a stiffening element to the balloon material, or the adhesion of one or more springs to the balloon.

The expansion member may include at least two coupled non-compliant balloons or two or more non-compliant balloon chambers within a single non-compliant balloon. The second non-compliant balloon or non-compliant balloon chamber may include an electrode wrapped around the second non-compliant balloon or non-compliant balloon chamber less than the circumference of the second non-compliant balloon or non-compliant balloon chamber.

In some embodiments, the expansion member includes a compliant balloon, such as a highly-compliant balloon. The compliant balloon may include longitudinal supports coupled to the compliant balloon such that longitudinal expansion of the expansion member may be limited. The expansion member may include one or more longitudinal supports with a length less than the length of the expansion member. The expansion member may include a compliant balloon with longitudinal supports in one or more discreet locations on the compliant balloon such as, for example, the distal end of the expansion member. The expansion member may include longitudinal supports such as, for example, overmolded fibers, variability in the hardness of materials included in the expansion member, variability in the thickness of the expansion member, or rib-type structures on the surface of the expansion member. Such support structures may, for example, allow circumferential expansion of the expansion member while simultaneously preventing longitudinal elongation.

In some instances, the ablation structure includes multiple separately wired or separately controlled longitudinal electrodes, longitudinal electrode zones, and/or longitudinal regions of varying widths. The ablation structure may include two or more actively coupled longitudinal electrodes or longitudinal electrode zones configured for simultaneous activation and deactivation. A first actively coupled longitudinal electrode or longitudinal electrode zone may be located in the first electrode position of the ablation structure and a second actively coupled longitudinal electrode or longitudinal electrode zone may be located in the last electrode position of the ablation structure such that end electrode regions of the ablation structure can be switched on or switched off in a simultaneously and/or coordinated fashion.

According to some embodiments of the disclosure, a method for treatment of tissue in body lumens with varying sizes is provided. The method generally includes inserting an ablation structure wrapped around an expansion member less than a circumference of the expansion member into a body lumen, expanding the expansion member to engage the ablation structure with a first portion of the body lumen less than a circumference of the body lumen, delivering energy through the ablation structure to the first portion of the body lumen less than the circumference of the body lumen, contracting the expansion member after delivering the energy to the ablation structure to the first portion of the body lumen, and rotating the ablation structure and expansion member with respect to the body lumen. In some instances, the ablation structure may be rotated about 180 degrees. The method may further include expanding the expansion member to engage the ablation structure with a second portion of the body lumen less than the circumference of the body lumen, and delivering energy through at least a portion of the ablation structure to the second portion of the body lumen less than the circumference of the body lumen. In some embodiments, delivering energy to the portion of the ablation structure to the second portion of the body lumen may include delivering energy to a subset of the number of longitudinal electrods or a subset of the number of the longitudinal zones. In certain instances, the method may further include selectively activating or deactiving one or more of the longitudinal electrodes or longitudinal zones.

In some instances, expanding the expansion member to engage the ablation structure with the first portion of the body lumen less than a circumference of the body lumen may include expanding at least the first balloon or a first chamber of the multi-chamber balloon. The first balloon or a portion of a surface surrounding the first chamber may be coupled with the ablation structure. In certain instances, expanding the expansion member to engage the ablation structure with the first portion of the body may include expanding at least the second balloon or a second chamber of the multi-chamber balloon to engage the ablation structure coupled with the expanded first balloon or the expanded first chamber.

The foregoing has outlined rather broadly the features and technical advantages of examples according to the disclosure in order that the detailed description that follows may be better understood. Additional features and advantages will be described hereinafter. The conception and specific examples disclosed may be readily utilized as a basis for modifying or designing other structures for carrying out the same purposes of the present disclosure. Such equivalent constructions do not depart from the spirit and scope of the appended claims. Features which are believed to be characteristic of the concepts disclosed herein, both as to their organization and method of operation, together with associated advantages will be better understood from the following description when considered in connection with the accompanying figures. Each of the figures may be provided for the purpose of illustration and description only, and not as a definition of the limits of the claims.

### BRIEF DESCRIPTION OF THE DRAWING

A further understanding of the nature and advantages of the embodiments may be realized by reference to the following drawings. In the appended figures, similar components or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.
FIG. 1A is a schematic diagram of a system for delivering treatment to a target treatment area including components configured according to various embodiments.
FIG. 1B is schematic diagram of one specific embodiment of the system shown in FIG. 1A.
FIG. 1C is a schematic diagram in perspective of one specific embodiment of the system shown in FIG. 1A.
FIG. 1D is a schematic diagram of a power source and a switching mechanism of the system shown in FIG. 1A and FIG. 1C.
FIG. 2 is a schematic view of portions of an upper digestive tract in a human.
FIG. 3A is a schematic view of an ablation device, in a compressed mode, within an esophagus.
FIG. 3B is a schematic view of an ablation device, in an expanded mode, within an esophagus.
FIG. 4 is a schematic view of an ablation device, in an expanded mode, coupled with multiple separately wired electrodes.
FIG. 5 is a schematic view of an ablation device, in an expanded mode, coupled with a single electrode segregated into separately wired electrode zones.
FIG. 6 is a schematic view of one specific embodiment of a torque break handle element with a detent feature.
FIG. 7 is a top cross-sectional view of circumferential ablation regions with an electrode of the ablation device of FIG. 2 divided into five selectable electrode regions.
FIG. 8 is a cross section perspective view of a uniform longitudinal electrode zone pattern of the ablation device of FIG. 2.
FIG. 9 is a cross section perspective view of a symmetrical longitudinal electrode zone pattern of the ablation device of FIG. 2.
FIG. 10 is a cross section perspective view of a symmetrical longitudinal electrode zone pattern with a large centered electrode of the ablation device of FIG. 2.
FIG. 11 is a cross section view of a linear electrode zone array pattern of the ablation device of FIG. 2.
FIG. 12 is a schematic view of the electrode patterns of the ablation device of FIG. 2.
FIG. 13 is a schematic view of balloon demarcations that may be used with the ablation device of FIG 2.
FIG. 14 is a schematic view of electrode wiring alignments that may be used with the ablation device of FIG 2.
FIG. 15A is a top cross sectional view of a dual-chambered non-compliant balloon of the ablation device in FIG. 2 folded in compressed mode.
FIG. 15B is a top cross sectional of a dual-chambered non-compliant balloon of the ablation device in FIG. 2 with the active chamber expanded and the passive chamber compressed.
FIG. 15C is a top cross sectional of a dual-chambered non-compliant balloon of the ablation device in FIG. 2 with the active chamber and passive chamber expanded.
FIG. 16 is a schematic view of the ablation device of Figure 2 including an expanded compliant balloon within an esophagus.
FIG. 17 is a schematic view of the ablation device of Figure 2 including longitudinal supports coupled to a compliant balloon within an esophagus.
FIG. 18 is a flow diagram illustrating a method for providing treatment to a target site area according to various embodiments.
FIG. 19 is a flow diagram illustrating a method for providing treatment to a target site area according to various embodiments.
FIG. 20 is a flow diagram illustrating a method for providing treatment to a target site area using an expansion member including a dual non-compliant balloon or a multi-chambered non-compliant balloon according to various embodiments.

### DETAILED DESCRIPTION

Methods, systems, and devices are described for providing treatment to a target site, such as a site within a body lumen. Systems may include an expansion member that may be coupled with a distal portion of a support shaft. An ablation structure with a circumference less than the circumference of the expansion member may be wrapped around the expansion member such that expanding the expansion member may engage body lumens of varying sizes.

The ablation structure may include a flexible circuit capable of bending with the expansion member upon which it may be disposed. Various aspects of flexible circuit may be similar to typical integrated circuits and microelectronic devices. The flexible circuit may include multiple separately wired or separately controlled longitudinal electrodes, longitudinal electrode zones, or both, such that each longitudinal electrode or longitudinal electrode zone may be selectively enabled or selectively disabled. In some instances, the ablation structure includes an electrode array, such as, for example, a bipolar electrode array. The ablation structure may include longitudinal electrodes of varying widths, longitudinal electrode zones of varying widths, or both.

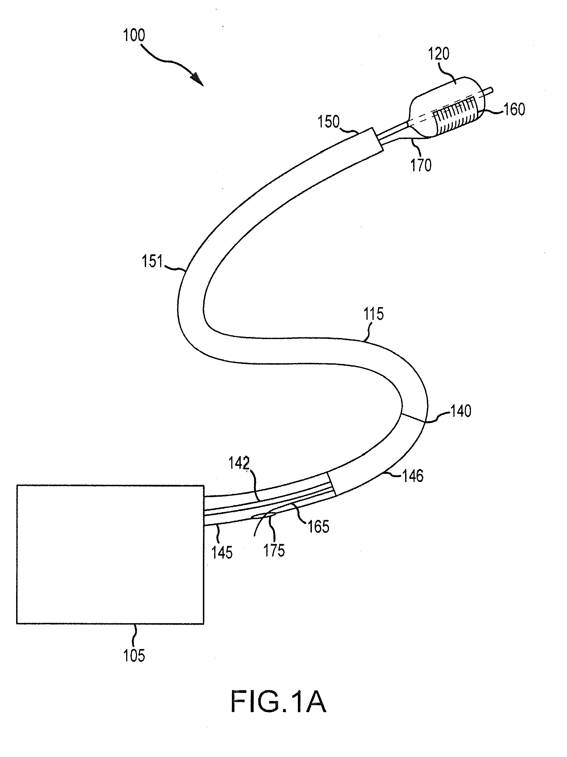

With reference to **FIG. 1A****,** a general system 100 for delivering treatment to a target treatment area is shown in accordance with various embodiments. The system 100 may be designed for providing treatment to a target site inside of a body, such as the wall of an organ or lumens in the gastrointestinal tract, for example. The system 100 may include a power source 105, a support shaft 115, a catheter 142, and/or an expansion member 120. The expansion member 120 may generally be configured to support an ablation structure 160 that may be used to supply therapy to the target site treatment area. The system 100 may operate by positioning a guide assembly 165 inside a body and passing the expansion member 120 over the guide assembly 165 such that the expansion member 120 may be delivered to a target site treatment area inside the body. The power source 105 may then be used to supply power to an ablation structure 160 disposed on the expansion member 120 so that therapy may be applied to the target site treatment area.

The expansion member 120 may be an inflatable device capable of transitioning between a compressed configuration and an expanded configuration with the use of supplementary expansion mechanisms. In some embodiments, the power source 105 is configured to inflate the expansion member 120. The collapsed configuration may be generally used when the expansion member 120 is inserted into the lumen and when re-positioned therein. When the expansion member 120 obtains a desired ablation positioning, the expansion member 120 may expand, such as by inflating from a deflated state (i.e. the compressed configuration) to a substantially inflated state (i.e., the expanded configuration).

The expansion member 120 may be configured to support an ablation structure 160. In some embodiments, the ablation structure is a therapeutic or diagnostic instrument, such as an ablation element that provides ablative energy to the target site treatment area. Some ablation structures 160 may be designed so that they make direct contact with a target site treatment area, including pressing of the ablation structure 160 against the target site.

The expansion member 120 may be coupled with the support shaft 115 such that the expansion member 120 may be maneuvered through a channel of the body, such as the esophagus, and at the target site treatment area. The support shaft 115 may include a proximal end 145 and a distal end 150, with the proximal end 145 configured to be coupled with the power source and inflation device 105 and the distal end 150 configured to be coupled with the expansion member 120. In some embodiments, the support shaft 115 includes an opening 175 configured to allow the entry and exit of the guide assembly 165 such that the catheter 142 is slidably movable relative to the guide assembly 165. The guide assembly entry point 175 may typically be located outside of the support shaft 115 and proximate the power source 105. In some embodiments, the support shaft 115 includes a break 140 that allows the distal portion of the support shaft 151 to rotate independently of the proximal portion of the support shaft 146. The break 140 may typically be located proximate the power source 105. Rotating the distal portion of the support shaft 151 may provide torque to the expansion member 120 and allow for better movement and control of the expansion member 120 at the target site treatment area. In some instances, the break 140 is enclosed within a protective container. The protective container may be configured to selectively rotate the distal portion of the catheter 142 independently of both the proximal portion of the catheter and the support shaft 151.

The power source 105 may generally provide power to the ablation structure 160 disposed on the expansion member 120. In some embodiments, power is provided from the power source 105 to the ablation structure 120 via one or more transmission lines 170 extending between the power source 105 and the expansion member 120 and housed within a channel of the support shaft 115.

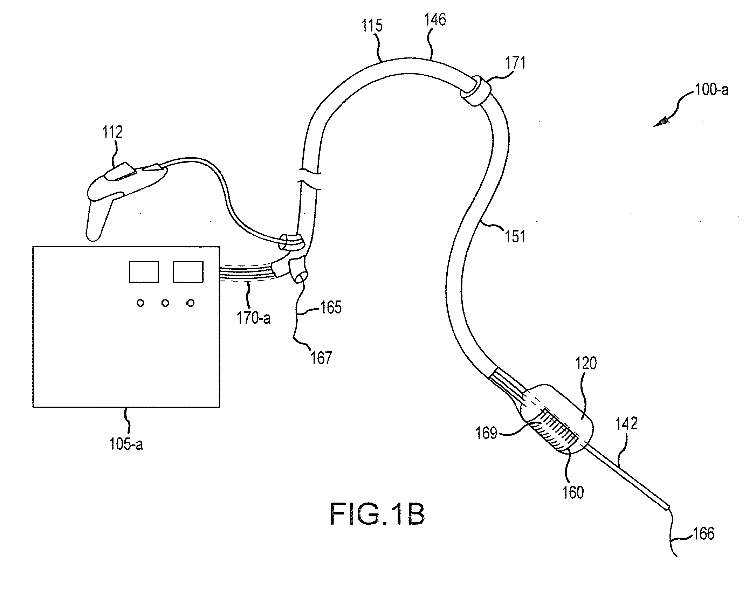

FIG. 1B illustrates a system 100-a that may be an example of the system 100 shown in FIG. 1A according to various embodiments. The system 100-a may include a generator 105-a, a hand-held air compressor 112, a guide assembly 165 with a distal end 166 and a proximal end 167, a support shaft 115, an expansion member 120, and/or an ablation structure 160 less than the circumference of the expansion member 120 coupled to the expansion member 120.

The expansion member 120 may include a balloon on which the ablation structure 160 may be supported. The expansion member 120 may be a flexible material capable of being curved or folded. The expansion member 120 may, when expanded, generally have an elongated cylindrical shape, including a rounded distal end. The expansion member 120 may taper at the proximal end and couple to the support shaft 115.

Disposed on a portion of the surface of the expansion member 120 may be an ablation structure 160 that may be configured to provide treatment to the target treatment area. As shown in FIG. **1B****,** the ablation structure 160 may include a single electrode or a series of electrodes 169 aligned adjacent to one another and that extend an arc length distance equal to or less than half the circumference of the expansion member 120. The one or more electrodes 169 may be interlaced, with approximately half of the electrodes extending from a first bus and approximately half of the electrodes extending from a second bus. The first bus or the second bus may be connected to a positive terminal and the other of the first bus or the second bus may be connected to a negative or ground terminal to thereby provide a bipolar electrode configuration. When connected to the power source 105-a, the one or more electrodes 169 may provide ablative energy to the target site treatment area.

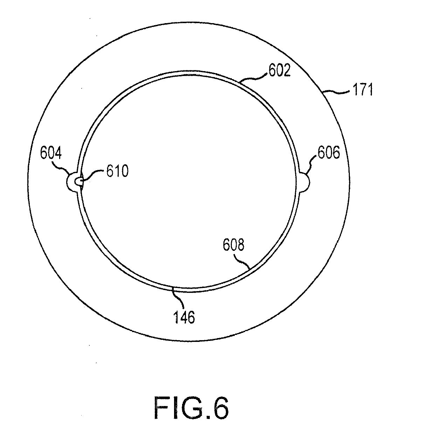

The expansion member 120 may be coupled with the support shaft 115. A set of transmission wires 170-a may extend from the power source 105-a to the expansion member 120 through the channel of the support shaft 115. The break 140 shown in FIG. 1A may serve as the dividing point between the distal portion 151 and proximal portion 146 of the support shaft 115, and may allow the distal portion 151 to rotate independently of the proximal portion 146. In some embodiments, the break 140 may be covered by a torque break handle element 171. The torque break handle element 171 may be made of any suitable material and may have any shape or size that allows it to cover the break 140 and protect the transmission lines 170. The torque break handle element 171 may have a generally cylindrical shape, although other shapes may be used. In some embodiments, the torque break handle element 171 is coupled with the distal portion of the support shaft 146 and is sufficiently long to extend over the break 140 and a portion of the proximal portion of the support shaft 151. In some embodiments, the torque break handle element 171 is decoupled from the proximal portion of the support shaft 146 so that the distal portion of the support shaft 151 may continue to rotate independently of the proximal portion of the support shaft 146. The coupling of the torque break handle element 171 to the distal portion of the support shaft 151 may allow the torque break handle element 171 to be configured to transmit rotational motion to the distal portion support shaft 151. In this manner, the torque break handle element 171 may also serve as a torque handle that aids a user in rotating the distal portion of the support shaft 151 to transmit torque to the expansion member 120. The torque break handle element 171 may also include a detent structure as described in **FIG. 6** such that the distal portion of the support shaft 151 may be rotated one or more defined distances, such as, for example, 180 degrees, and remain fixed in one or more defined rotational positions.

The use of a non-circumferential ablation structure to ablate a circumferential area may generally include one or more repositioning actions to ablate the circumferential area. If the circumference of a non-circumferential ablation structure is unequal to half the circumference of the body lumen being treated, then the repositioning and subsequent ablation may result in an overlap of the ablation structure with previously ablated areas. In some embodiments, electrode regions overlapping previously ablated regions of the body lumen may be selectively switched off, and/or electrode regions not overlapping previously ablated regions of the body lumen may be selectively switched on.

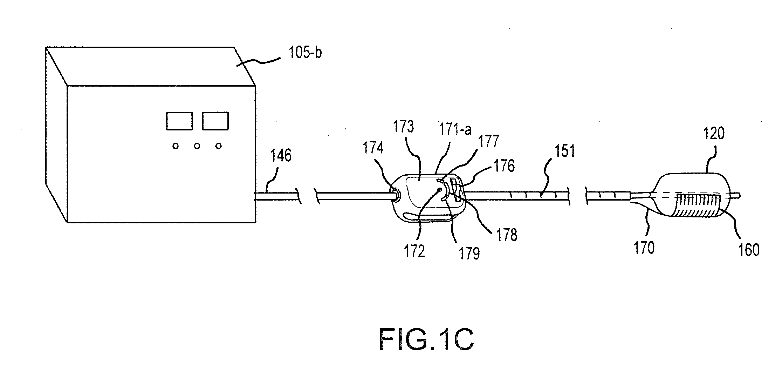

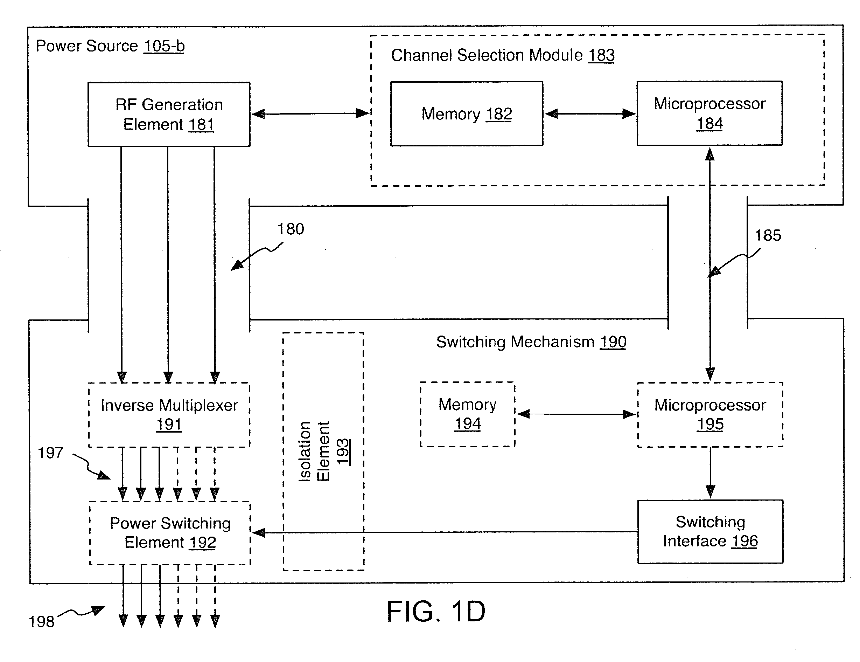

Referring now to **FIG. 1C****,** in some embodiments, the break 140 is enclosed in a torque break handle element 171-a. The torque break handle element 171-a may include an opening configured for insertion of the guide wire 165 (See **FIG. 1**). The torque break handle element 171-a may be configured to selectively rotate the distal portion of the catheter 142 independently of both the proximal portion of the catheter and the support shaft 151. The torque brake handle element 171-a may include a switching mechanism 190 (see **FIG.** 1D) enclosed in a protective housing 173. A switching interface such as, for example, a button 172 may be coupled to the switching mechanism 190, the button configured to select which longitudinal electrode regions are enabled. In certain cases, each button press event selects one of a series of predefined electrode region activation configurations. Visual indicators such as, for example, led lights 177, 178, 179 may be attached to the housing 173 and coupled to the switching mechanism 190, configured to provide an indication of which electrode regions are selectively enabled. In a three-region electrode implementation, for example, a first button press may activate all three electrode regions, a second button press may activate only both flanking regions, a third button press may activate only the center region and one flanking region, and a fourth button press event may activate only the center region and the opposing flanking regions. The series may repeat for subsequent button press events. It will be appreciated by one skilled in the art that many other configurations are possible. Certain implementations may include other manual selection interfaces such as, for example, a multiple button interface.

Still referring to **FIG. 1C****,** the interface between the distal portion of the support shaft 151 (see FIG. 1B), the torque break handle element 171-a, and the proximate portion of the support shaft 146 is shown in perspective. The torque break handle element 171-a may be coupled to both the distal and proximal portions of the support shaft 146, 151 such that the torque break handle element 171-a does not rotate in relation to the support shaft 115. A catheter rotation interface such as, for example, a flywheel 176 may be coupled to all or part of the outer circumference of the catheter and partially protrude through an opening in the housing 173 such that an operator may rotate the distal end of the catheter 142 by rotating the flywheel 176. The flywheel mechanism may include detents (not shown) to arrest rotation at one or more pre-defined rotational orientations. The rotation of the flywheel 176 may transmit torque and/or rotation to the expansion member at about a one to one torque ratio, thus repositioning the center of the ablation structure 120 in accordance with the pre-defined rotational orientation.

In some embodiments, a switching mechanism is configured to switch on and switch off longitudinal electrode regions, thus controlling the active width of the ablation structure and consequently the arc length of the ablation region at the treatment site. With reference now to **FIG. 1D****,** a power source 105-b is coupled to a switching mechanism 190. An RF generation element 181 may generate and transmit RF energy across one or more channels 180. In some cases, the number of defined longitudinal electrode regions may be less than or equal to the number of RF channels 180 supported by the power source 105-b, with each defined longitudinal electrode region coupled to a single RF channel 180. In such a configuration, the switching interface 196 may be communicatively coupled with a channel selection module 183 integrated with the power source 105-b. The channel selection module may include a microprocessor 184 and a memory 182. The switching interface may be an analog interface or a digital interface, and may additionally be coupled with a microprocessor 195 and a memory 194. The switching mechanism 190 may communicate operator selections of electrode regions to the channel selection module 183 which then either enables or disables the RF channel 180 associated with each electrode region in accordance with the received operator selections.

Additionally, or alternatively, the power source may be configured to transmit RF energy across one or more channels concurrently or in a defined sequence independent of any operator switching selections. In some embodiments, the switching mechanism switches RF output channels 180 on or off by blocking the transmission from the RF generation element 181. The switching interface 196 may be communicatively coupled with a power switching element 192 such as, for example, a metal-oxide-semiconductor field-effect transistor or a relay. The switching interface may be an analog interface or a digital interface, and may additionally be coupled with a microprocessor 195 and a memory 194. In some instance, an isolation element 193 is positioned between the power switching element 192 and the switching interface 196, logic element 195 and memory 194. The switching interface 196 communicates operator selections of longitudinal electrode regions to the power switching element 192 which then either blocks or allows RF transmission in accordance with the operator selections, thus enabling or disabling the longitudinal electrode region associated with the RF channel.

In some instances, the switching mechanism 190 monitors current and/or interprets other signals communicated from the power source 105-b to determine, in part, when to switch a channel on or off. Additionally, or alternatively, the power source 105-b may control the switching behavior of the switching mechanism 190 via a one-way or two-way communication channel 185 coupling the power source logic element 184 and the switching mechanism logic element 195. In certain implementations, the power source 105-b may receive feedback from the switching mechanism 190, such as, for example, an acknowledgment that switching instructions were received and/or that the directed switching behavior was executed. Communication between the logic elements 184, 195 may implement an established communication protocol such as, for example, I2C or SPI.

In some instances, longitudinal electrode regions are not associated with particular RF generation element output channels 180. The RF generator 181 may be configured to transmit RF energy on one or more output channels to the power switching element 192 where such power switching element 192 then reroutes the RF energy to multiple longitudinal electrode regions in accordance with operator selections.

In certain implementations, the number of defined electrode regions exceeds the number of RF channels supported by the power source 105-b. For example, an RF generation element 181 may support a maximum of 3 RF channels, where the ablation structure 160 (see **FIG. 1**) may include 6 separately-wired electrode regions. In such cases, the RF generator 181 may be configured to transmit RF energy on only one output channel to the power switching element 192, where such power switching element 192 then reroutes the RF energy to multiple longitudinal electrode regions. Alternatively, the RF generation element 181 may be configured to transmit RF energy over multiple output channels to an inverse multiplexer 191, where such inverse multiplexer 191 expands the number of channels by, for example, re-routing the common return of the bipolar system.

As an example, an operator may determine the body lumen size at a treatment site visually or through the use of a sizing device. The operator may then insert the ablation device in the body lumen and position the ablation structure at the treatment site. Electrode regions may be selected such that the partial circumferential ablation region is half or slightly more than half of the circumference of the treatment site. A first ablation may be performed, followed by a 180 degree rotation of the ablation structure. A second ablation may be performed with the electrode region selection unchanged, resulting in a full 360 degree ablation with reduced ablation overlap.

Additionally, or alternatively, an operator may determine body lumen size at the treatment site visually or through the use of a sizing device. The operator may then insert the ablation device in the body lumen and position the ablation structure at the treatment site. A first ablation may be performed where all electrode regions are enabled, followed by a 180 degree rotation of the ablation structure. The operator may then visually inspect the treatment site to determine the appropriate electrode regions to selectively enable in order achieve complete circumferential ablation with reduced ablation overlap. This visual inspection may be done by, for example, endoscopic visualization. A second ablation may be performed with the electrode region selection made by the operator in accordance with the visual inspection. This can result in a full 360 degree ablation with reduced ablation overlap. In some instances, more than two rotational repositioning steps may be performed. For example, the operator may treat a lumen more than 2 times the arc length of the ablation structure. In this situation, the operator may perform a first ablation selectively enabling all electrode regions, then rotate the ablation structure 120 degrees and perform a second ablation with all electrode regions enabled. The operator may then rotate the ablation structure another 120 degrees and visually inspect the treatment site to determine the appropriate electrode regions to selectively enable for the third ablation such that ablation overlap is minimized.

In addition to the use of visual indicators, in some embodiments, other methods may be used to assist in the identification and selection of longitudinal electrode regions. In some embodiments, the power source 105-b includes instructions configured to execute a sizing algorithm to determine the circumference of the lumen. This determined value may be used to retrieve an ablation sequence from a lookup table associating one or more lumen circumferential measurements with one or more ablation sequences. This table may be stored in memory 182. The channel selection module 183 may direct the RF generation element 181 to execute the obtained ablation sequence without regard to any operator selections. Additional computer software, such as image analysis software, could be used to identify previously ablated regions as part of an algorithm to identify, select, and enable longitudinal electrode regions.

The ablation of tissue may result in a variation to the impedance of that tissue as compared to unablated tissue. A probe sensor may also be used to determine the size of the non-ablated regions of the circumferential treatment site by comparing the impedance of the region defined by the second placement position of the ablation structure with previous impedance data from the first ablation. This data may then be used to select the longitudinal electrode regions to be enabled. It will be appreciated by one skilled in the art that these and other automated selection algorithms may be implemented on one or more communicatively coupled computer devices external to the power source 105-b.

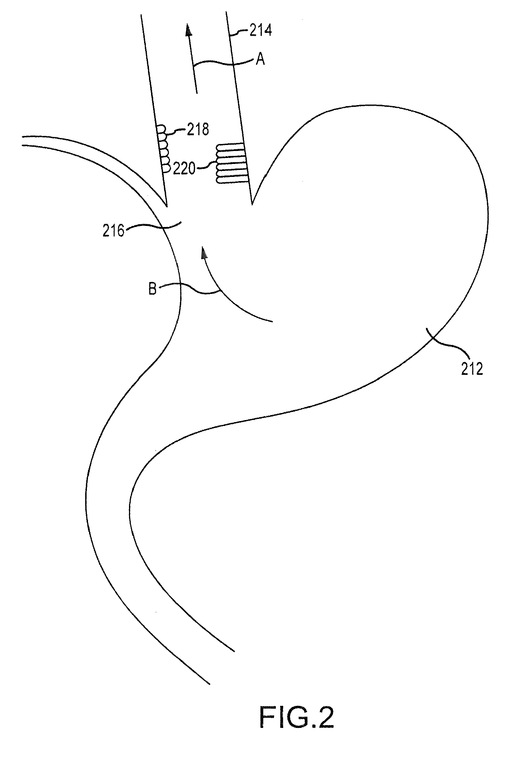

Referring now to **FIG. 2****,** certain disorders may cause the retrograde flow of gastric or intestinal contents from the stomach 212, into the esophagus 214, as shown by arrows A and B. Although the causes of these problems are varied, this retrograde flow may result in secondary disorders, such as Barrett's esophagus, which require treatment independent of and quite different from treatments appropriate for the primary disorder-such as disorders of the lower esophageal sphincter 216. Barrett's esophagus is an inflammatory disorder in which the stomach acids, bile acids and enzymes regurgitated from the stomach and duodenum enter into the lower esophagus causing damage to the esophageal mucosa. When this type of retrograde flow occurs frequently enough, damage may occur to esophageal epithelial cells 218. In some cases the damage may lead to the alteration of the squamous cells, causing them to change into taller specialized columnar epithelial cells 220. This metaplastic change of the mucosal epithelium from squamous cells to columnar cells is called Barrett's esophagus. Although some of the columnar cells may be benign, others may result in adenocarcinoma.

In some embodiments, the methods, systems, and devices described are configured to treat columnar epithelium of selected sites of the esophagus through the ablation of the tissue. The term "ablation" as used herein means thermal damage to the tissue causing tissue or cell necrosis. It will be appreciated by one skilled in the art that some therapeutic procedures may have a desired treatment effect that falls short of ablation, such as, for example, some level of agitation or damage that is imparted to the tissue to insure a desired change in the cellular makeup of the tissue, rather than necrosis of the tissue. In some instances, a variety of different energy delivery devices may be utilized to create a treatment effect in a superficial layer of tissue, while preserving intact the function of deeper layers, as described hereafter.

Cell or tissue necrosis may be achieved with the use of energy, such as RF energy, at appropriate levels to accomplish ablation of mucosal or submucosal level tissue, while substantially preserving muscularis tissue. Such ablation may be utilized to remove the columnar growths 220 from the portions of the esophagus 214 so affected.

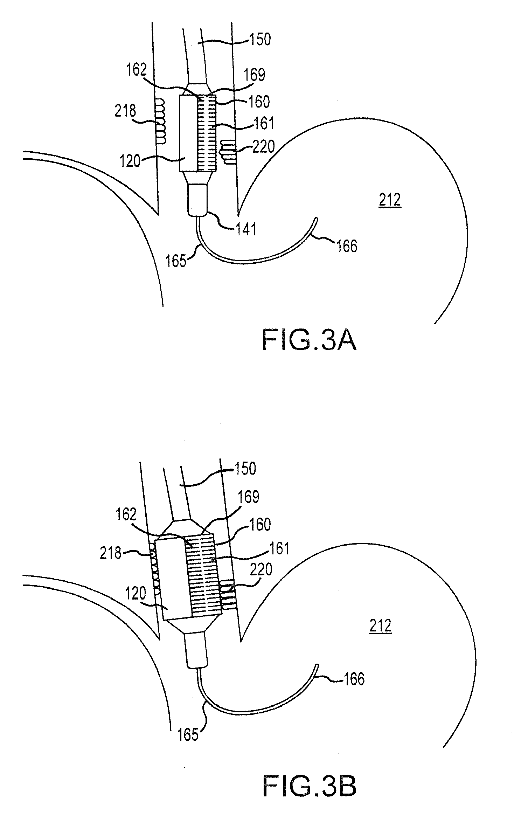

Referring now to **FIG. 3A** and **FIG. 3B****,** the expansion member 120 may be inserted into the body in any of various ways including, for example, guide assembly 165 placement, endoscopic placement, surgery, or by other means. Expansion member 120 may be an example of expansion member 120 of FIG. 1A, FIG. 1B, and/or FIG. 1C. Referring now to **FIG. 3A****,** the expansion member 120 is shown in a compressed configuration in accordance with various embodiments. The expansion member 120 may be configured for transitioning between the compressed configuration shown and an expanded configuration shown in **FIG. 3****B.** In the expanded configuration, at least one dimension of the expansion member 120 may have increased. In various embodiments, the expanded configuration is significantly larger than the collapsed configuration and allows the expansion member 120 to contact a treatment surface 220. The ablation structure 160 may be delivered to the treatment site area within the body lumen while in a compressed state. This low-profile configuration may allow for ease-of-access to the treatment site without discomfort or complications to the patient. When an endoscope (not shown) is used, the distal end of the support shaft 151 may be positioned along the outside of the endoscope. Alternately, an endoscope may be used to visualize the pathway that expansion member 120 should follow during placement. The distal end of a guide assembly 166 may be positioned along the outside of an endoscope and left in the body lumen after removal of the endoscope. The proximal end of the guide assembly 167 may be inserted into the distal end of the catheter 141 and the catheter 142 inserted into esophagus following the path determined by the guide assembly 165.

An ablation structure 160 is provided and may be coupled to the expansion member 120 and positioned at the distal end of the support shaft 151. In some instances, the expansion member 120 is bonded to the distal end of the support shaft 151. The ablation structure may include one or more electrodes 169. The one or more electrodes 169 may include multiple longitudinal electrodes zones 161, 162 of equal or varying widths. The one or more electrodes 169 may be coupled to a power source 105 (see *e.g.* FIG. 1A) configured for powering the one or more electrodes and/or longitudinal electrode zones 161, 162 at levels appropriate to provide the selectable ablation of tissue to a predetermined depth of tissue.

In some embodiments, the ablation structure 160 includes a flexible, non-distensible backing. For example, the ablation structure 160 may include a thin, rectangular sheet of polymer materials such as polyimide, polyester or other flexible thermoplastic or thermosetting polymer film. The ablation structure 160 may also include polymer covered materials, or other nonconductive materials. Additionally, the backing may include an electrically insulating polymer, with an electro-conductive material, such as copper, deposited onto a surface so that an electrode pattern may be etched into the material to create an electrode array.

The ablation structure 160 may be operated in direct contact with, the tissue wall of the treatment site. This may be achieved by coupling the ablation structure 160 to an expansion member 120, which has a configuration that may be expandable in a shape that conforms to the dimensions of the inner lumen of the treatment site, such as the human lower esophageal tract. An expansion member 120 may include, for example, a balloon, such as a compliant balloon and/or a balloon with a tapered geometry that expands to an expanded configuration when inflated.

In some embodiments, selective enabling of one or more longitudinal electrodes 169 and/or longitudinal electrode zones 161, 162 regulates and controls the amount of energy transferred to the tissue at a tissue site such as the inner wall of a lumen. The ablation structure 160 may extend an arc length distance equal to or less than half the circumference of the expansion member 120. When the expansion member 120 expands, the expansion member 120 adapts to the circumference of the body lumen while the ablation structure adapts to less than the circumference of the lumen. The ablation structure 160 may distribute a constant electrode element density per unit area across an arc length less than the circumference of the body lumen.

The ablation structure 160 may be positioned and repositioned such that energy may be selectively applied to all or a portion of the inner circumference of the lumen where treatment may be desired. This may be accomplished by first positioning the expansion member 120 at the treatment area in a compressed configuration. Once the ablation structure 160 is advanced to the appropriate treatment site, expansion member 120 may be inflated, which advances the ablation structure 160 to engage the internal wall of the body lumen. The desired treatment energy may then be delivered to the tissue at the treatment site according to selective enablement of one or more longitudinal electrodes and/or longitudinal electrode zones 161, 162.

In certain embodiments, the ablation structure 160 may deliver a variety of different types of energy including but not limited to, radio frequency, microwave, ultrasonic, resistive heating, chemical, a heatable fluid, optical including without limitation, ultraviolet, visible, infrared, collimated or non collimated, coherent or incoherent, or other light energy, and the like.

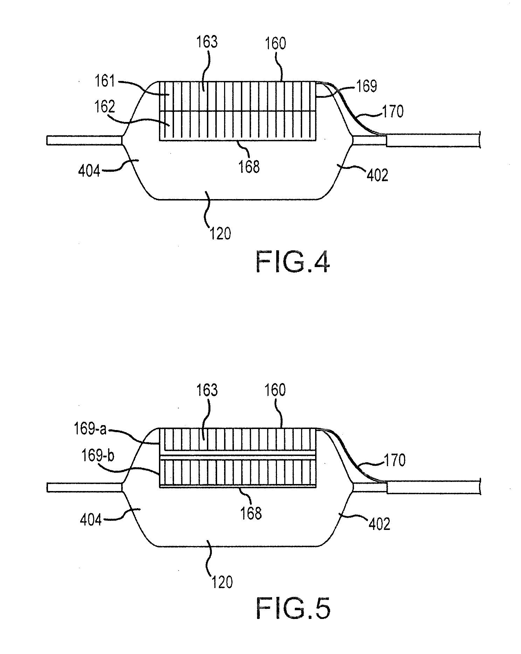

Referring now to **FIG. 4** and **FIG. 5****,** the ablation structure 160 may generally extend from the proximal end of the expansion member 402 to the distal end of the expansion member 404. In some embodiments, the ablation structure 160 may be positioned between the tapered ends of the ablation structure. The ablation structue 160 may be an example of the ablation structure 160 of FIGs. 1A and/or 1B, for example. The ablation structure 160 may be located on a surface of the expansion member 120 and may provide the expansion member 120 sufficient structure such that the ablation structure 160 may be transported along the guide assembly 165 without crumpling upon itself. The ablation structure 160 may also provide apposition force when the expansion member 120 may be deflected against a target treatment area, such as tissue at a treatement site.

Referring now to **FIG. 4****,** in some embodiments, the ablation structure 160 includes a single electrode 169 with multiple longitudinal electrode zones 161, 162. Longitudinal electrode zones 161, 162 may be selectively enabled via multiple transmission lines 170 extending between the power source 105 (see, *e.g.,*

**FIGs.** 1) and the longitudinal electrode zones 161, 162. Ablation structure 160 has an electrode array 163 etched on its surface, and may be aligned between the distal 404 and proximal 402 ends of the expansion member 120. In some embodiments, the expansion member 120 includes a passive area adjacent to the lateral edges of the ablation structure 168.

Referring now to **FIG. 5****,** in some embodiments, the ablation structure 160 includes multiple longitudinal electrodes 169-a, 169-b. Longitudinal electrodes 169-a, 169-b may be selectively enabled via multiple transmission lines 170 extending between the power source 105 (see, *e.g.,* **FIGs.** 1) and the longitudinal electrodes 169-a, 169-b. Ablation structure 160 has an electrode array 163 etched on its surface, and may be aligned between the distal 404 and proximal 402 ends of the expansion member 120. In some embodiments, the expansion member 120 includes a passive area adjacent to the ablation structure edges 168.

Referring now to **FIG. 6****,** the interface between the distal portion of the support shaft 151 (see FIG. 1B), the torque break handle element 171, and the proximate portion of the support shaft 146 is shown in cross section. The proximal portion of the support shaft 146 and torque break handle element 171 may be an example of the proximal portion of the support shaft 146 and torque break handle element 171 of FIG. 1B. The torque break handle element 171 may be coupled to the distal portion of the support shaft 151 such that the torque break handle element 171 may be prevented from rotating relative to the distal portion of the support shaft 151. An inner circumference 602 of the torque break handle element 171 may be defined by 2 round or semi round concave portions 604, 606 spaced 180 degrees apart from each other along the inner circumference 602 of the torque break handle element 171. An outer circumference 608 of the proximate portion of the support shaft 146 fits snuggly within the inner circumference 602 of the torque break handle element 171 such as to form a protective coupling. An outer circumference 608 of the proximate portion of the support shaft 146 may be defined by a retractable domed member 610 having a round or semi-round cross section. The retractable domed member 610 of the proximate portion of the support shaft 146, when aligned with either of the concave portions 604, 606 along the inner circumference 602 of the torque break element 171, prevents rotation of the torque break element 171 relative to the proximate portion of the support shaft 146 when less than a specified rotational force may be applied to the proximate portion of the support shaft 146 relative to the protective element, or vice versa.

The retractable domed member 610 of the proximate portion of the support shaft 146 may be biased by a resilient member (not shown), such as a spring for example, such that when the domed member 610 aligns with either of the concave portions 604, 606 along the inner circumference 602 of the protective element 171, the retractable domed member 610 engages the concave portion 604 or 606 and prevents rotation of the proximate portion of the support shaft 146 relative to the torque break handle element 171. When a rotational force may be applied to the proximate portion of the support shaft 146 relative to the torque break handle element 171 greater than a biasing force of the resilient member, the domed member 610 will retract allowing the proximate portion of the support shaft 146 to rotate with respect to the torque break handle member 171 in either direction, for example, until the domed member 610 aligns with the other concave portion 604 or 606 180 degrees from a starting position. In this way, the distal portion of the support shaft 151 may rotate precisely 180 degrees relative to the proximate portion of the support shaft 146 with a simple twisting motion. The rotation of the distal portion of the support shaft 151 may transmit torque and/or rotation to the expansion member at about a one to one torque ratio, thus repositioning the center of the ablation structure 160 180 degrees counter to the prior ablation structure 160 position.

The use of a non-circumferential ablation structure to ablate a circumferential area may generally include one or more repositioning actions to ablate the circumferential area. If the circumference of a non-circumferential ablation structure is unequal to half the circumference of the body lumen being treated, then the repositioning and subsequent ablation may result in an overlap of the ablation structure with previously ablated areas. In some embodiments, electrode regions overlapping previously ablated regions of the body lumen may be selectively switched off, and/or electrode regions not overlapping previously ablated regions of the body lumen may be selectively switched on.

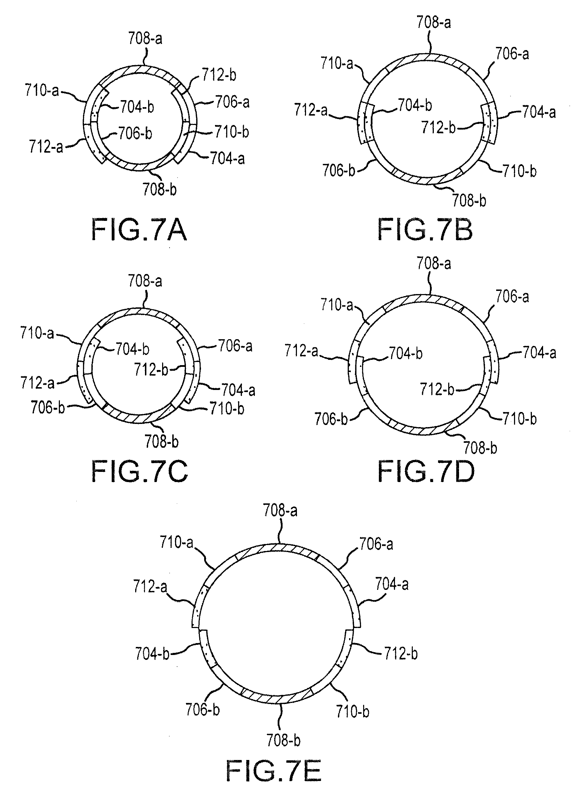

Referring now to **FIG. 7A** through **FIG 7E****,** two-placement circumferential ablation patterns of body lumens of varying diameters using a non-circumferential ablation structure are shown. In some embodiments, an ablation structure may include electrode regions consisting of multiple electrodes, or alternatively, a single electrode segregated into multiple longitudinal electrode zones arranged to ablate associated regions of a body lumen 704, 706, 708, 710, 712. Electrode regions may be an example of the single electrode 169 segregated into multiple electrode zones 161, 162 of FIG. 1C and FIG. 4. Additionally, or alternatively, electrode regions may be an example of multiple longitudinal electrodes 169-a, 169-b of FIG. 5. In some embodiments, a center longitudinal electrode region with a width greater than any other longitudinal electrode region in the set of multiple longitudinal electrodes or multiple longitudinal electrode zones may be flanked by a symmetrical configuration of regions of lesser width. A circumferential ablation is shown relating to two ablation structure placements. At the first ablation structure placement, all longitudinal electrode regions are enabled for a period of time, ablating the associated lumen regions 704-a, 706-a, 708-a, 710-a, 712-a defined by all longitudinal electrodes or longitudinal electrode zone. A single 180 degree rotation of the ablation structure results in the ablation structure obtaining a second placement. The degree to which the second placement overlaps the lumen regions ablated by the first placement is dependent, in part, on the diameter of the body lumen.

Referring now to **FIG. 7A****,** for certain lumen diameters, the flanking electrode regions are positioned such that their associated ablation regions 704-b, 706-b, 710-b, 712-b fully overlap previously ablated regions 704-a, 706-a, 710-a, 712-a. With reference now to **FIG. 7B****,** for certain other lumen diameters, electrode regions are positioned such that their associated ablation regions 704-b, 712-b fully overlap previously ablated regions 704-a, 712-a. Enabling the electrode regions associated with the overlapping ablation regions 704-b, 712-b may over-ablate the previously ablated lumen regions 704-a, 712-a. In some embodiments, the overlapping electrode regions are not enabled, thus eliminating over-ablation of the overlapped ablation regions 704-a, 706-a, 710-a, 712-a, while fully ablating the unablated region 708-b.

Referring now to FIG. 7C, for certain other lumen diameters, the flanking electrode regions are positioned such that some associated ablation regions 704-b, 712-b fully overlap previously ablated regions 706-a, 710-a, and other associated ablation regions 706-b, 710-b partially overlap previously ablated regions 704-a, 712-a. Enabling the electrode regions associated with the overlapping ablation regions may over-ablate all or part of the previously ablated regions 704-a, 706-a, 710-a, 712-a. In some embodiments, the outer-most flanking electrodes are not enabled, thus eliminating over-ablation of the overlapped ablation regions 706-a, 710-a, and the inner-most flanking electrodes are not enabled, thus reducing over-ablation of the overlapped ablation regions 704-a, 712-a, while fully ablating the unablated region 708-b.

With reference now to **FIG. 7D****,** for certain other lumen diameters, the flanking electrode regions are positioned such that the associated ablation regions 704-b, 712-b partially overlap previously ablated regions 704-a, 712-a. Enabling the electrode regions associated with the overlapping ablation regions may over-ablate portions of previously ablated regions 704-a, 712-a. In some embodiments, the outer-most flanking electrode regions are not enabled, thus reducing over-ablation of the overlapped regions 704-a, 712-a, while fully ablating the unablated region 708-b.

With reference now to **FIG. 7E****,** for lumen diameters equal to twice the arc length of the ablation structure, the flanking electrode regions may be positioned such that no associated ablation regions 704-b, 706-b, 708-b, 710-b, 712-b overlap any previously ablated regions 704-a, 706-a, 708-a, 710-a, 712-a. All electrode regions are enabled for both the first ablation structure placement and the second ablation structure placement without any over-ablation of any previously ablated regions 704-a, 706-a, 708-a, 710-a, 712-a.

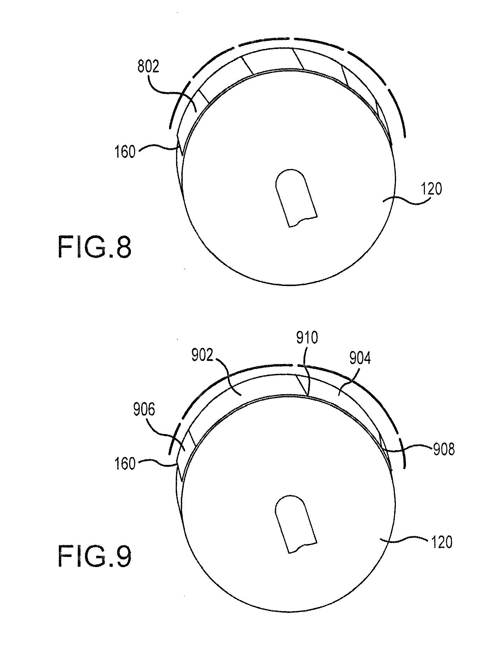

In some embodiments, an ablation structure includes a large single electrode segregated into multiple longitudinal electrode zones of either uniform or varying widths configured to reduce the degree of ablation-region overlap and thus reduce the degree of over ablation. Referring now to **FIG. 8****,** in some embodiments, an ablation structure 160 less than the circumference of the expansion member 120 may include a single electrode segregated into adjacent longitudinal electrode zones of uniform width 802. The ablation structures 160 of FIG. 8 through FIG. 10 may be examples of the ablation structure of FIG. 1A and/or FIG. 1B. In certain instances, narrow width electrode segregations 802 may be implemented such that the degree of overlap, and thus over ablation, may be further reduced by switching off one or more flanking longitudinal electrode zones. Additionally, or alternatively, an ablation structure with uniform or varying width electrode regions may be implemented with multiple electrodes.

Other alternative longitudinal electrode zone patterns may be implemented such that over ablation resulting from overlapping ablation regions may be reduced, such as, for example, variations of symmetrical longitudinal electrode configurations. Referring now to **FIG. 9** a simple bilaterally symmetrical configuration is shown. In some embodiments, an equal number of longitudinal electrode zones are positioned on both sides and adjacent to the center point of the length of the electrode such that the electrode zones are ordered from largest to smallest starting from the two center-most electrodes zones. For example, two large longitudinal electrode zones 902, 904 may be positioned on either side of and adjacent to the center point of an electrode 910. A smaller longitudinal electrode zone 906, 908 may be positioned adjacent to the opposing edge of each of the two larger longitudinal electrode zones 902, 904. The degree of overlap, and thus over ablation, may be further reduced by switching off one or more flanking longitudinal electrode zones. Additionally, or alternatively, an ablation structure with uniform or varying width electrode regions may be implemented with multiple electrodes.

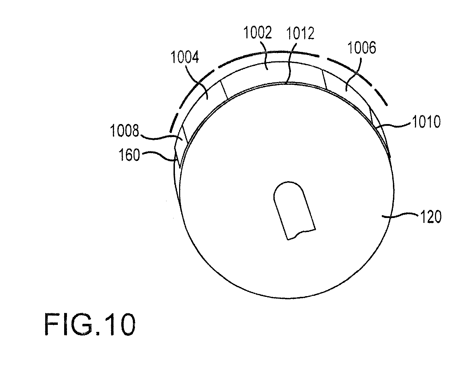

In certain instances, a center longitudinal electrode zone with a length greater than any other longitudinal electrode zone on the electrode in the set of multiple longitudinal electrode zones is flanked by a symmetrical configuration of longitudinal electrode zones of lesser length. Referring now to **FIG. 10****,** two longitudinal electrode zones 1004, 1006, 1008, 1010 are positioned on each side and adjacent to the centered larger longitudinal electrode zone 1002. The combined arc length of the two outer-most smaller longitudinal electrode zones 1008, 1010 may be equal to the combined arc length of the two inner-most smaller longitudinal electrode zones 1004, 1006, and equal to the arc length of the centered larger longitudinal electrode zone 1002. The degree of overlap, and thus over ablation, may be further reduced by switching off one or more flanking longitudinal electrode zones. Additionally, or alternatively, electrode region configurations may be implemented with multiple electrodes.

Two or more longitudinal electrode zones may be electrically coupled such that the coupled set of longitudinal electrode zones may be enabled and disabled simultaneously from a single switching mechanism over a single wire or channel. In some embodiments, the two outer-most longitudinal electrode zones are electrically coupled to one another, and the two inner-most longitudinal electrode zones are also electrically coupled to one another. The power source 105 (see, *e.g.,* **FIG. 2**) may include an automated and/or manual switching mechanism configured to enable and disable the electrically coupled electrode zones. In some embodiments, a separate switching mechanism receives one or more transmission lines 170 from the power source 105. The separate switching mechanism may act as an inverse multiplexer, expanding the number of channels of a power source, thus potentially increasing the number of possible longitudinal electrode zones. Additionally, or alternatively, one or more electrically coupled electrode regions may be implemented with multiple electrodes.

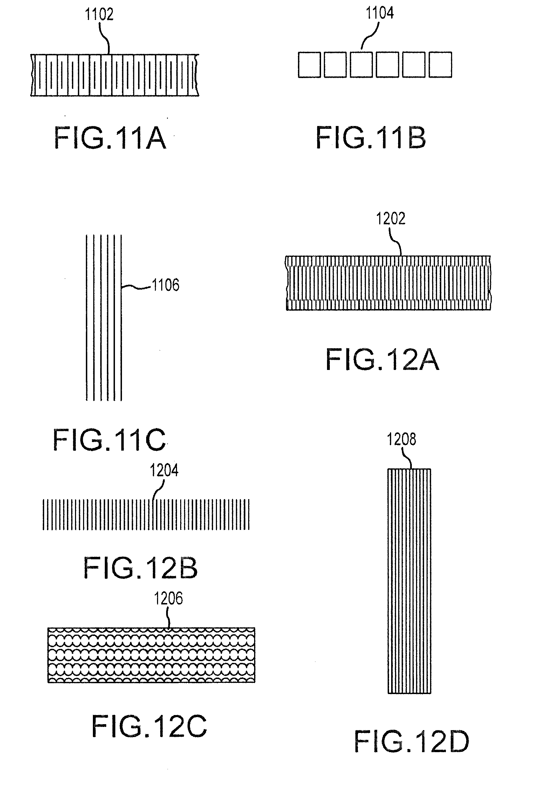

Referring now to **FIG. 11A** through **FIG. 11C****,** the electrode patterns may be varied depending on the length of the site to be treated, the depth of the mucosa and submucosa, in the case of the esophagus, at the site of treatment, and other factors. The electrode patterns 1102 - 1208, may be examples of electrode patterns included with the electrode array 163 of FIG. 4 and FIG. 5. An electrode array pattern may be composed of particular electrode elements that may be arranged in various configurations, such as, for example, a circumferential orientation or a longitudinal orientation. An electrode element is a conductive element of an electrode array. In some instances, electrode elements may be aligned parallel to one another. The density of the electrode elements may affect the depth of an ablation treatment. The longitudinal electrode or longitudinal electrode zone patterns may be aligned in an axial or transverse direction across the one or more electrodes, or formed in a linear or non-linear parallel matrix or series of bipolar pairs or monopolar electrode.

One or more different patterns may be coupled to various locations of the ablation structure 160. For example, an electrode array, as shown in **FIG. 11A** through **FIG. 11C****,** may comprise a pattern of bipolar axial interlaced finger electrodes 1102, six bipolar rings 1104 with 2 mm separation, or monopolar rectangles 1104 with 1 mm separation. Other suitable RF electrode patterns may be used including, without limitation, those patterns shown in **FIG. 12A** through **FIG. 12D****.** Patterns may include, for example, bipolar axial interlaced finger electrodes with 0.3 mm separation 1202, monopolar bands with 0.3 mm separation 1204, bipolar rings with 0.3 mm separation 1206, and/or undulating electrodes with 0.2548 mm separation 1208.

The depth of treatment may be controlled by the selection of appropriate treatment parameters by the operator as described in the examples set forth herein. One parameter that may affect the depth of treatment may be the density of electrode elements. As the spacing between electrode elements decreases, the depth of treatment of the affected tissue also decreases. Very close spacing of the electrode elements may limit the current and resulting ohmic heating to a shallow depth such that injury and heating of the submucosal layer are minimized. For treatment of esophageal tissue using RF energy, it may be desirable to have a spacing between adjacent electrode elements be no more than, (i) 3 mm, (ii) 2 mm, (iii) 1 mm (iv) 0.5 mm or (v) 0.3 mm (vi) 0.1 mm and the like.

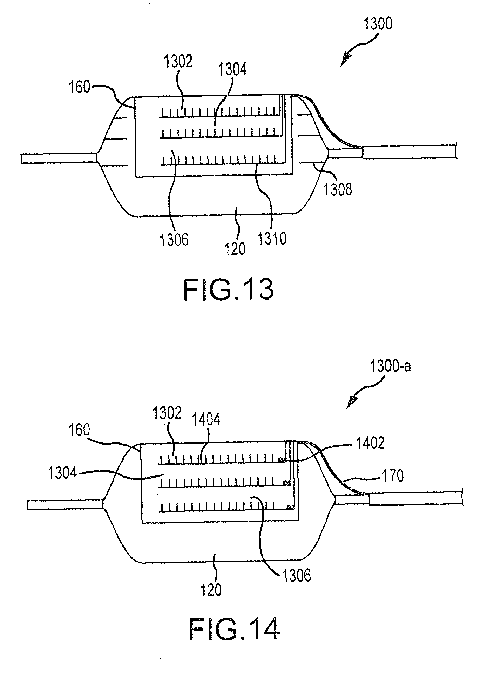

After the second placement of the ablation structure in a two-placement circumferential ablation procedure, unablated regions are identified and the corresponding longitudinal electrodes and/or electrode zones are selected for enablement. In some embodiments, the unablated regions of the treatment area are visually compared to the longitudinal electrode regions. Referring now to **FIG. 13****,** the distal portion of the system 1300 may include visual indicators configured to assist in the visual identification of the longitudinal electrode regions. The expansion member 120 may be an example of the expansion member 120 of FIGs. 1A, 1B, 4 and/or 5. In some embodiments, the ablation structure 160 is attached to the expansion member 120. The ablation structure 160 includes multiple longitudinal electrode regions 1302, 1304, 1306. The expansion member may include visual indicators 1308 such as, for example, printed lines or painted lines, aligned in-line with the boundaries of the longitudinal electrode regions 1310. With reference to **FIG. 14****,** the traces 1402 connecting the transmission lines 170 with the ablation structure 160 may be aligned in-line with the boundaries of the longitudinal electrode regions 1404. The ablation structue 160 may be an example of the ablation structure 160 of FIGs. 1A, 1B, 4 and/or 5. These and other visual indicators may act as a clue to aid the operator in positioning and/or verifying the position of the ablation structure 160 and the alignment of the longitudinal electrode regions 1302, 1304, 1306 with the edges of unablated lumen regions of the circumferential treatment site.

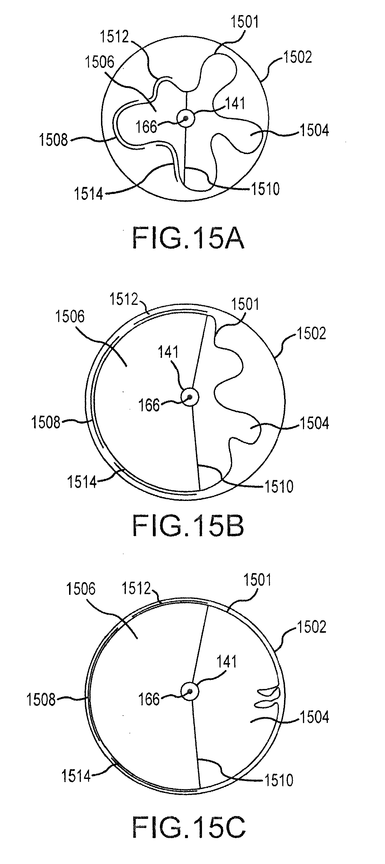

In some embodiments, the expansion member 120 (see *e.g.,* FIGs. 1) includes one or more non-compliant balloons 1501 made from a material such as, for example, polyurethane. The expansion member 120 may be an example of the expansion member 120 of FIGs. 1A and/or 1B. Referring now to **FIG. 15A****,** in some embodiments, the expansion member 120 includes a non-compliant balloon 1501 with two chambers 1504, 1506 separated by a non-permeable barrier 1510. In an alternate embodiment, the expansion member includes two non-compliant balloons. An ablation structure 160 may be attached to the surface of one of the chambers 1506, the active chamber, such that all longitudinal electrode regions 1508, 1512, 1514, are associated with the active chamber 1506. A passive chamber 1504 may be defined by the absence of any longitudinal electrode regions 1508, 1512, 1514. The expansion member 120 may be inserted into the lumen 1502 according to the method described previously. With reference now to **FIG. 15B****,** when the expansion member 120 obtains the desired first placement, the active chamber 1506 may be fully expanded by, for example, the power source 105 or the hand-held compressor 112 (see *e.g.* **FIG. 1B****)** such that all longitudinal electrode regions are fully deployed. Referring now to **FIG. 15C****,** once the active chamber 1506 may be fully inflated, the passive chamber 1504 may be then inflated until the surface of the passive chamber 1504 engages the ablation structure 160 with sufficient pressure to force the longitudinal electrode regions 1508, 1512, 1514 to engage the interior surface of the lumen 1502. If the circumference of the interior of the lumen 1502 may be less than the circumference of the expansion member, the passive chamber may not fully inflate.

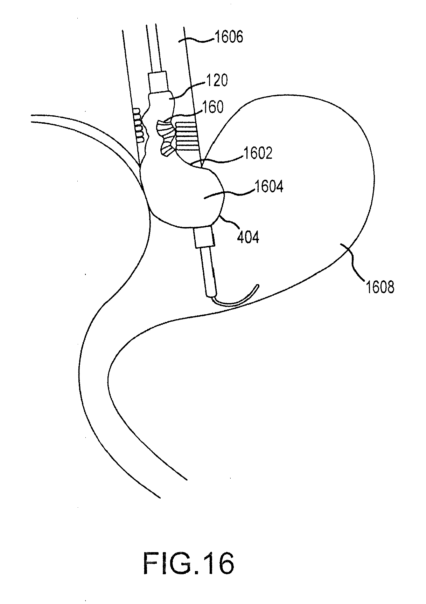

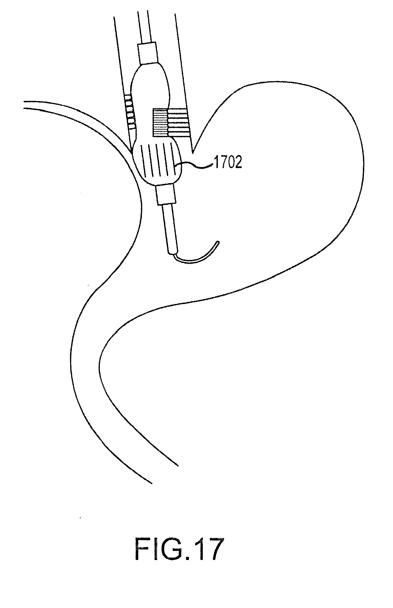

Referring now to **FIG. 16****,** in some embodiments, the expansion member 120 includes a balloon 1602 made from highly compliant material such as, for example, silicone. The expansion member 120 may be an example of the expansion member 120 of FIGs. 1A, 1B, 1C, 4, 5, 13, and/or 14. In the absence of any structural constraints, the passive area 1604 of the highly compliant balloon 1602 may hyperinflate. For example, if the distal end of the expansion member 404 extends into a chamber 1608 past the lumen 1606, the passive section 1604 may hyper-inflate, which may result in improper apposition of the ablation structure 160 and uneven engagement of the treatment area. With reference now to **FIG. 17****,** in some embodiments, the highly compliant balloon may be co-molded in two durometers configured to reduce strain placed on the compliant material in unconstrained areas. The balloon may include narrow rib-like structures 1702 along a portion of the length of the balloon or along the entire length of the balloon configured to allow circumferential stretch. An alternate embodiment includes overmolded fibers and/or a similar composite structure configured to constrain the degree of longitudinal stretch.

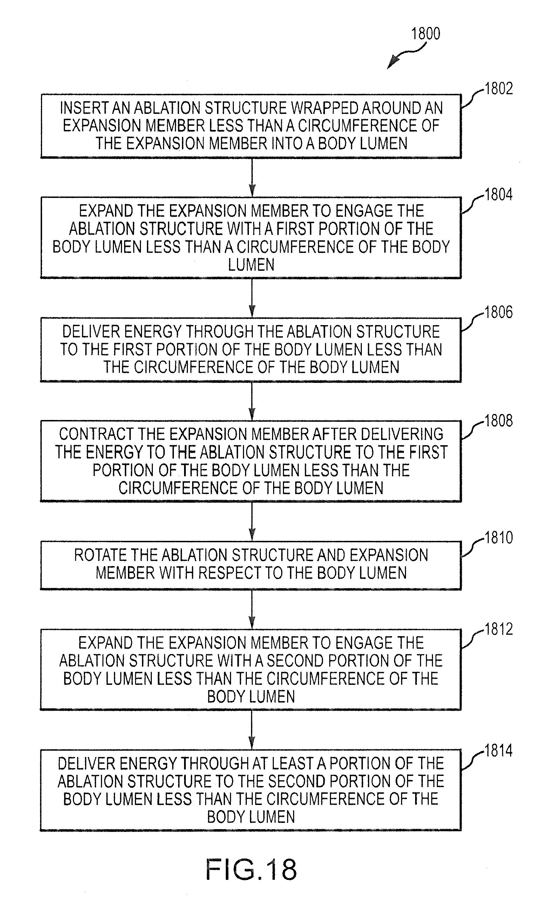

With reference to **FIG. 18** a general method 1800 of using various embodiments of the systems and/or devices described herein is shown in accordance with various embodiments. For example, method 1800 may be implemented utilizing the various embodiments of system 100, expansion member 120, ablation structure 160, torque break handle element 171, and/or other devices and/or components. At block 1802, the ablation structure 160 less than the circumference of the expansion member 120 and the expansion member 120 are inserted into the body lumen. A guide assembly 165 may be used such that the expansion member 120 may be passed over the guide assembly 165 delivering the ablation structure 160 to a target treatment area inside the body lumen.

At block 1804, the expansion member 120 may be expanded such that the ablation structure 160 engages a first part of a circumferential treatment area of the body lumen less than the circumference of the body lumen. In some instances, the expansion member 120 includes a highly-compliant balloon. In some embodiments, the power source 105 and/or the hand-held compressor 112 may be used to expand the expansion member 120.

At block 1806, energy may be delivered through the ablation structure 160 to first part of a circumferential treatment area of the body lumen less than the circumference of the body lumen. In some embodiments, the ablation structure 160 includes two or more longitudinal electrodes or longitudinal electrode zones of varying widths. In some embodiments, the ablation structure 160 includes two or more longitudinal electrodes or longitudinal electrode zones configured to be selectively enabled or selectively disabled.

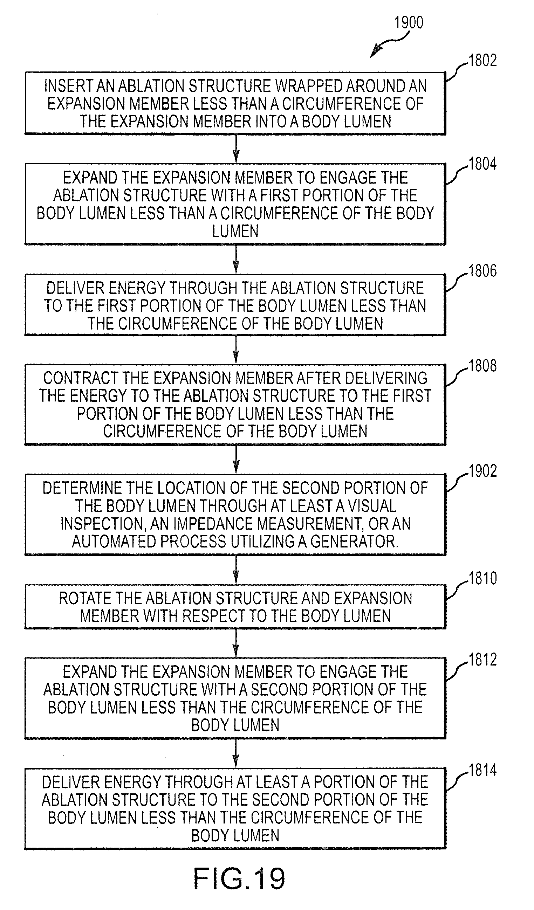

With reference now to **FIG. 19****,** at block 1902, in some embodiments, a second location of a portion of the body lumen may be determined through one or more methods, such as, for example, visual inspection of the lumen tissue and system 100, measuring impedance of tissue before and after ablation, or by an automated process that uses a power source such as a generator. For example, method 1900 may be implemented utilizing the various embodiments of visual indicators 1308 of FIG. 13 and 1402 of FIG. 4. Method 1900 may be an example of method 1800 of **FIG. 18****.**

With reference again to **FIG. 18****,** at block 1808, after delivering energy to a first part of a circumferential treatment area, contracting the expansion member 120 such that the expansion member may be configured to more easily move in the body lumen. At block 1810, the ablation structure 160 and expansion member 120 are rotated with respect to the body lumen and a second position may be obtained different from the position obtained for the first ablation. In some embodiments, the degree of rotation is about 180 degrees. The torque break handle element 171 may be used to effect the 180 degree rotation.

At block 1812, upon obtaining a second position, the expansion member 120 may be expanded such that the ablation structure 160 may be engaged with a second portion of the circumferential treatment area of the body lumen less than the circumference of the body lumen. In some embodiments, the ablation structure 160 includes two or more longitudinal electrodes or longitudinal electrode zones configured to be selectively enabled or selectively disabled.

At block 1814, energy may be delivered through the ablation structure 160 to the second part of a circumferential treatment area of the body lumen less than the circumference of the body lumen. In some instances, less than the total number of longitudinal electrodes or longitudinal electrode zones are selectively switched on and/or off. In certain cases, selective activation switching of longitudinal electrodes or longitudinal electrode zones may be performed in a manner appropriate to ablate all or a portion of the unablated circumferential treatment area. Other steps may also be utilized in accordance with various embodiments.

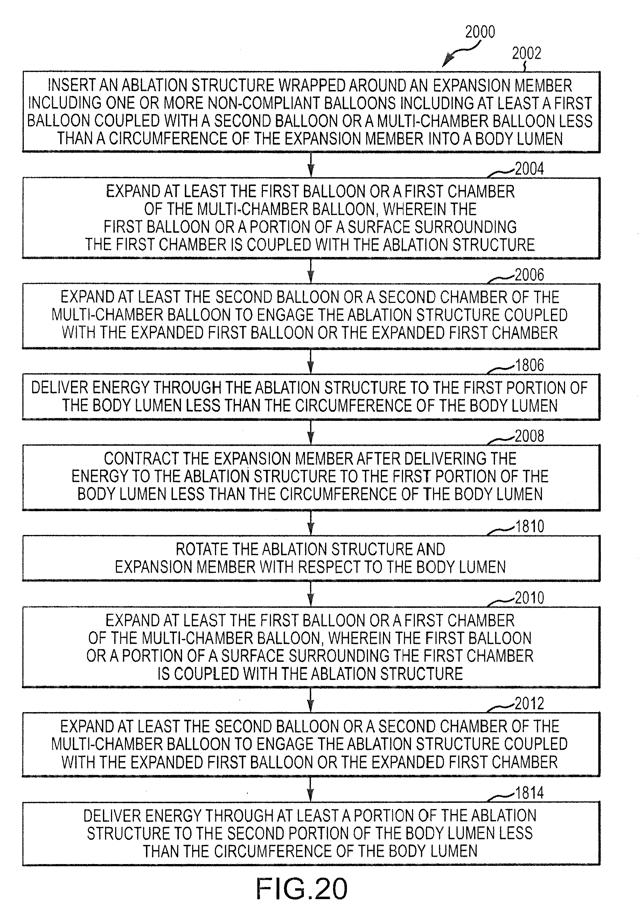

With reference to **FIG. 20** a general method 2000 of using various embodiments of the systems and/or devices described herein is shown in accordance with various embodiments. For example, method 2000 may be implemented utilizing the various embodiments of system 100, expansion member 120, one or more non-compliant balloons 1501, ablation structure 160, torque break handle element 171, and/or other devices and/or components.

At block 2002, the ablation structure 160 less than the circumference of the expansion member 120 and the expansion member 120 are inserted into the body lumen. A guide assembly 165 may be used such that the expansion member 120 may be passed over the guide assembly 165 delivering the ablation structure 160 to a target treatment area inside the body lumen. In some embodiments, the expansion member 120 includes a first balloon coupled to a second balloon. In another embodiment, the expansion member 120 includes a multi-chambered balloon, such as, for example, a dual-chambered balloon. In certain instances, the ablation structure 160 is configured to fold in a manner that avoids the folding in and/or pinching of the longitudinal electrodes or longitudinal electrode zones.

At block 2004, the first balloon or the first chamber of the multi-chambered balloon may be expanded. In some embodiments, the first balloon is coupled with the ablations structure 160. In another embodiment, a portion of the first chamber is coupled with the ablation structure 160. The first balloon or the first chamber may be expanded such that the ablation structure 160 is fully deployed. In some embodiments, the power source 105 and/or the hand-held compressor 112 may be used to expand the first balloon or the first chamber of the multi-chambered balloon.

At block 2006, the second balloon or the second chamber of the multi-chambered balloon may be expanded. The second balloon or the second chamber may be expanded until the surface of the second balloon or second chamber engages the interior surface of the lumen with sufficient pressure to force the longitudinal electrode regions to engage the interior surface of the lumen. If the circumference of the interior of the lumen 1502 may be less than the circumference of the expansion member, the second balloon or the second chamber may not fully expand. In certain instances, longitudinal supports are coupled with the expansion member to limit longitudinal expansion of the expansion member. In some embodiments, the power source 105 and/or the hand-held compressor 112 may be used to expand the first balloon or the first chamber of the multi-chambered balloon.

At block 1806, energy may be delivered through the ablation structure 160 to first part of a circumferential treatment area of the body lumen less than the circumference of the body lumen. In some embodiments, the ablation structure 160 includes a two or more longitudinal electrodes or longitudinal electrode zones of varying widths. In some embodiments, the ablation structure 160 includes a two or more longitudinal electrodes or longitudinal electrode zones configured to be selectively enabled or selectively disabled. In certain instances, the ablation structure 160 includes a bipolar electrode array.

At block 2008, after delivering energy to a first part of a circumferential treatment area, contracting the expansion member 120 such that the expansion member 120 may be configured to more easily move in the body lumen. At block 1810, the ablation structure 160 and expansion member 120 are rotated with respect to the body lumen and a second position may be obtained different from the position obtained for the first ablation. In some embodiments, the degree of rotation is about 180 degrees. The torque break handle element 171 may be used to effect the 180 degree rotation.

At block 2010, the first balloon or the first chamber of the multi-chambered balloon may be expanded. In some embodiments, the first balloon is coupled with the ablations structure 160. In another embodiment, a portion of the first chamber is coupled with the ablation structure 160. The first balloon or the first chamber may be expanded such that the ablation structure 160 may be fully deployed. In some embodiments, the power source 105 and/or the hand-held compressor 112 may be used to expand the first balloon or the first chamber of the multi-chambered balloon.

At block 2012, the second balloon or the second chamber of the multi-chambered balloon may be expanded. The second balloon or the second chamber may be expanded until the surface of the second balloon or second chamber engages the interior surface of the lumen with sufficient pressure to force the longitudinal electrode regions to engage the interior surface of the lumen. If the circumference of the interior of the lumen 1502 may be less than the circumference of the expansion member, the second balloon or the second chamber may not fully expand. In some embodiments, the power source 105 and/or the hand-held compressor 112 may be used to expand the first balloon or the first chamber of the multi-chambered balloon.

At block 1814, energy may be delivered through the ablation structure 160 to the second part of a circumferential treatment area of the body lumen less than the circumference of the body lumen. In some instances, less than the total number of longitudinal electrodes or longitudinal electrode zones are selectively switched on and/or off. In certain cases, selective activation switching of longitudinal electrodes or longitudinal electrode zones may be performed in a manner appropriate to ablate all or a portion of the unablated circumferential treatmen area. Other steps may also be utilized in accordance with various embodiments.

The foregoing description provides examples, and is not intended to limit the scope, applicability or configuration of the various embodiments. Rather, the description and/or figures provide those skilled in the art with an enabling description for implementing various embodiments. Various changes may be made in the function and arrangement of elements.

Thus, various embodiments may omit, substitute, or add various procedures or components as appropriate. For instance, it should be appreciated that the methods may be performed in an order different than that described, and that various steps may be added, omitted or combined. Also, aspects and elements described with respect to certain embodiments may be combined in various other embodiments. It should also be appreciated that the following systems, methods, and devices, may individually or collectively be components of a larger system, wherein other procedures may take precedence over or otherwise modify their application.

The foregoing descriptions of specific embodiments have been presented for purposes of illustration and description. They are not intended to be exhaustive or to limit the invention to the precise forms disclosed, and obviously many modifications and variations are possible in light of the above teaching. The embodiments were chosen and described in order to explain the principles of the various embodiments and its practical application, to thereby enable others skilled in the art to utilize the various embodiments with various modifications as are suited to the particular use contemplated. It is intended that the scope of the various embodiments be defined by the Claims appended hereto and their equivalents.

## Claims

1. An ablation device for treatment of tissue in body lumens with varying sizes comprising:
a support shaft;
an expansion member coupled with a distal portion of the support shaft;
an ablation structure comprising a plurality of longitudinal electrode regions, wherein:
the ablation structure is wrapped around the expansion member less than a circumference of the expansion member; and
the ablation structure and the expansion member are configured to engage at least a portion of the body lumens with the varying sizes.

2. The ablation device of claim 1, wherein the longitudinal electrode regions comprise a plurality of longitudinal electrodes, wherein at least one of the longitudinal electrodes is configured to be selectively enabled or disabled.

3. The ablation device of claim 1, wherein the longitudinal electrode regions comprise a plurality of longitudinal electrode zones, wherein at least one of the longitudinal electrode zones is configured to be selectively enabled or disabled.

4. The ablation device of claim 2 or 3, wherein the plurality of longitudinal regions comprise at least two longitudinal regions with different widths.

5. The ablation device of any preceding claim, wherein the ablation structure comprises a bipolar electrode array.

6. The ablation device of any preceding claim, wherein the expansion member comprises one or more non-compliant balloons.

7. The ablation device of claim 6, wherein the ablation structure is configured to fold with respect to the non-compliant balloon to avoid pinching the ablation structure.

8. The ablation device of claim 6, wherein the one or more non-compliant balloons comprises at least a first balloon coupled with a second balloon or a multi-chamber balloon.

9. The ablation device of any one of claims 1 to 5, wherein the expansion member comprises a compliant balloon.

10. The ablation device of claim 9, further comprising a plurality of longitudinal supports coupled with the expansion member to limit longitudinal expansion of the expansion member.

11. The ablation device of any preceding claim, wherein the ablation structure is configured to allow at least one of the electrode regions to be selectively disabled and to allow at least one of the electrode regions to be selectively enabled so that ablation energy is deliverable from a subset of the longitudinal electrode regions or only part of the longitudinal electrode regions.

12. The ablation device of any preceding claim, wherein the plurality of longitudinal electrode regions comprise longitudinal electrode regions that are separately wired and/or separately controllable to allow at least one electrode region to be selectively enabled and selectively disabled.

13. The ablation device of any preceding claims, comprising a switching mechanism, which may be user operable, configured to enable and disable longitudinal electrode regions, thereby controlling an active width of the ablation structure and consequently an arc length of an ablation region at a treatment site.

14. The ablation device of any preceding claim, wherein the expansion member and the ablation structure are rotatable relative to a proximal portion of the support shaft.
